# EUROPEAN PATENT SPECIFICATION

(11) **EP 3 796 880 B1**
(45) Date of publication and mention of the grant of the patent: **04.12.2024**
(21) Application number: 19806425.5
(22) Date of filing: 07.05.2019
(51) Int. Cl.: A61B 13/00, A61B 90/16, A61B 90/18, A61N 5/10

(54) **IMMOBILIZATION SYSTEM HAVING BITE-BLOCK STABILIZATION AND METHOD OF USING SAME**
IMMOBILISIERUNGSSYSTEM MIT AUFBISSSTABILISIERUNG UND VERFAHREN ZUR VERWENDUNG DAVON
SYSTÈME D'IMMOBILISATION COMPORTANT UNE STABILISATION DE BLOC DE MORSURE ET SON PROCÉDÉ D'UTILISATION

(30) Priority: 25.05.2018 US 201815989379
(43) Date of publication of application: 31.03.2021
(73) Proprietor: Klarity Medical Products, LLC, Newark OH 43055 (US)
(72) Inventor: CALHOUN, Matthew P., Heath, Ohio 43056 (US); DEVAULT, Mary Ellen, Newark, Ohio 43055 (US)
(74) Representative: Peters, Andreas
(86) International application number: PCT/US2019/031036
(87) International publication number: WO 2019/226332

(56) References cited:
- EP-A1- 3 003 199
- WO-A1-2013/173477
- FR-A1- 2 919 171
- US-A- 3 236 235
- US-A- 3 774 616
- US-A- 5 066 231
- US-A- 5 590 643
- US-A1- 2002 108 616
- US-A1- 2007 004 993
- US-A1- 2009 095 790
- US-A1- 2010 095 968
- US-A1- 2014 007 868
- US-A1- 2016 095 739
- US-A1- 2016 095 739
- ANONYMOUS: "CIVCO RADIOTHERAPY. Head & Neck Immobilization Training Video", 27 September 2016 (2016-09-27), XP054980966, Retrieved from the Internet <URL:https://www.youtube.com/watch?v=gU-5!peuU98&feature=youtu.be> [retrieved on 20190626]

## Description

### TECHNICAL FIELD

The present disclosure relates generally to an immobilization system, in particular, for making an anatomically high-accuracy moldable immobilization system having a bite-block stabilization feature, well-suited for patient immobilization during radiotherapy or other applications requiring a high degree of accuracy in patient positioning. This is of particular importance for radiotherapy of the head and neck region.

### BACKGROUND OF THE INVENTION

The present invention relates to an immobilization system, for persons whose bodies or body parts are required to be retained in a particular position or attitude. In particular, a need exists for a simple and easy to use immobilization system that allows repetitive patient positioning with a high degree of accurate repeatability across time, for example, wherein radiation treatments must be directed at a certain anatomical point across different times. Such precise positioning of the head and neck area, as might be required for radiotherapy of that region, is particularly problematic due to difficulty in immobilizing the area, while maintaining the patient's ability to breath, and with a reasonable degree of patient comfort.

The use of low-temperature thermoplastics for patient positioning is well-known and dates back to splinting devices invented in the 1960's (Larson, USPN 5,540,876). Splints are heated, usually in hot water, to a temperature of about 160° F, whereupon they become pliable and can be molded by hand directly on the patient's body part. These devices are well known in the field of occupational therapy and include splints with padding or cushioning material laminated to the thermoplastic to provide comfort against the patient's skin.

Plastic materials have been successfully used in the past for making splints, casts and the like. USPN 3,490,444 describes the use of thermoplastic polydienes like transpolyisoprene and transpolychloroprene, which melt between 140° F (60° Celsius) and 212° F (100° Celsius), and which harden by crystallization at about 140° F (60° Celsius), such that these plastics can be formed for use as a body supporting member. Poly (epsilon-caprolactone) (PCL) has also been found to be an excellent splint or cast material (USPN 4,144,223). Polyurethanes based on prepolymers of poly (epsilon-caprolactone) have also been used (USPN 4,316,457).

As described in earlier patents, the polymers can be heated in hot water at a temperature usually exceeding 122° F (50° Celsius) and up to about 212° F (100° Celsius), whereby they become soft, self-adherent and sufficiently pliable to be deformed and shaped as a cast, splint or protective device. When allowed to cool in air to about 140° F (40° Celsius), the materials will remain pliable, moldable and cohesive for a period of several minutes, exhibiting a hysteresis, as described in USPN 3,490,444. During this time the splint, cast or device can be molded directly to the patient without discomfort, and the shaped plastic sets hard by crystallization to assume a rigid form as a useful body support member or protective device.

In the field of radiation therapy, precise patient positioning is essential for treatment accuracy. An additional requirement is that patients be precisely re-positioned for repeated radiation treatments. Repositioning to an accuracy within millimeters, or even less, is generally desirable. This requires the positioning to be reproduced accurately each time the patient undergoes a treatment. Low-temperature thermoplastic masks in conjunction with other positioning methods are often used for such positioning. Masks are heated to a temperature of about 160° F (71° C), and formed directly on to the patient's head or other body part. The masks may be affixed to a table supporting the patient and cooled to form a firm mask holding the patient steady for treatment. After treatment, the mask may be removed. When the patient returns for the next treatment, the mask is releasably reattached, holding the patient in a reproduced position for treatment.

Various masks are used for radiation therapy treatments, including stereotactic head masks holding the top and bottom of the patient's head (Vilsmeier, USPN 5,702,406). In the field of moldable head and neck masks, a particular problem is holding the mask, when heated, to conformance around the patient's facial and head area for the short period of time required for the mask to cool and assume a stable shape. A tension exists between holding the mask accurately to conform to these features, while maintaining a reasonable degree of patient comfort.

Another goal of patient immobilization systems for radiation therapies is the avoidance of as much mass surrounding the patient's body as possible, as the mass tends to attenuate a radiotherapy beam. Therefore, the avoidance of unnecessary bulk in the immobilization system confers an advantage over more bulky systems. However, at the same time, systems have found it difficult to achieve adequate stabilization without using relatively high-mass immobilization devices.

US2002/108616A1, FR2919171A1, US2014/007868A1, US2016/095739A1, WO2013/173477A1 and US5066231A further disclose relevant information.

### SUMMARY OF THE INVENTION

The disclosed invention relates to methods to for making an immobilization system. Especially for those systems intended for use on the human head and neck area, there may be a retention mask, molded to the face or head contours of the patient, holding the desired body parts firmly to the formable support. This allows for high reproducibility in positioning.

The system includes a bite block immobilization system having a bite block with a dental interface releasably engageable with a plurality of teeth of a patient. The dental interface may be supplied with the system, or may be added at the time of use by a user. The dental interface releasably engages the teeth of a patient and allows very highly accurate repositioning during multiple procedures. In some embodiments, the bite block may have an integral bite block airway passage, allowing a patient to have unobstructed breathing both during molding of the retention mask and during subsequent use of the system.

In the invention, the bite block releasably cooperates at a tongue diverter receiver with a tongue diverter, that may, by way of example only and not limitation, be used to divert the tongue away from a therapeutic radiation beam. Unclaimed steps for employing the system are further described below.

The invention is defined by appended independent claim 1, a preferred embodiment is defined by appended dependent claim 2.

### BRIEF DESCRIPTION OF THE ILLUSTRATIONS

Without limiting the scope of the as disclosed herein and referring now to the drawings and figures:
FIG. 1 is an unassembled elevated perspective view of an embodiment of the current specification;
FIG. 2 is an unassembled elevated perspective view of a detail of a portion of the embodiment of FIG. 1;
FIG. 3 is an assembled elevated perspective view of a further detail of a portion of the embodiment of FIG. 1;
FIG. 4 is a further elevated perspective view of a detail of a portion of the embodiment of FIG. 1;
Fig. 5 is a lateral elevation view of an assembled embodiment of the device of FIG. 1 seen being placed in operating position between a patient's mandible and maxilla;
FIG. 6 is an elevated perspective view of an embodiment of the device of FIG.1 in place in an operating position between a patient's mandible and maxilla;
FIG. 7 is an elevated perspective view of a prior-art facial positioning mask placed over the embodiment of the device of FIG. 1 in an operating position;
FIG. 8 is a further embodiment of the device of FIG. 1, further showing a digital manipulator in place in a non-operating position;
FIG. 9 is a further embodiment of the device of FIG. 1, further showing a digital manipulator in place in an operating position; and
FIG. 10 is a further embodiment of the device of FIG. 1, showing a release tool in a disengaged state relative to a bite block and tongue diverter; and
FIG. 11 is a further embodiment of the device of FIG. 1, showing a release tool in an engaged state relative to a bite block, having released a tongue diverter.

These illustrations are provided to assist in the understanding of the exemplary embodiments of the method of forming an immobilization system having a bite block stabilization feature and materials related thereto described in more detail below and should not be construed as unduly limiting the specification. In particular, the relative spacing, positioning, sizing and dimensions of the various elements illustrated in the drawings may not be drawn to scale and may have been exaggerated, reduced or otherwise modified for the purpose of improved clarity. Those of ordinary skill in the art will also appreciate that a range of alternative configurations have been omitted simply to improve the clarity and reduce the number of drawings.

### DETAILED DESCRIPTION OF THE INVENTION

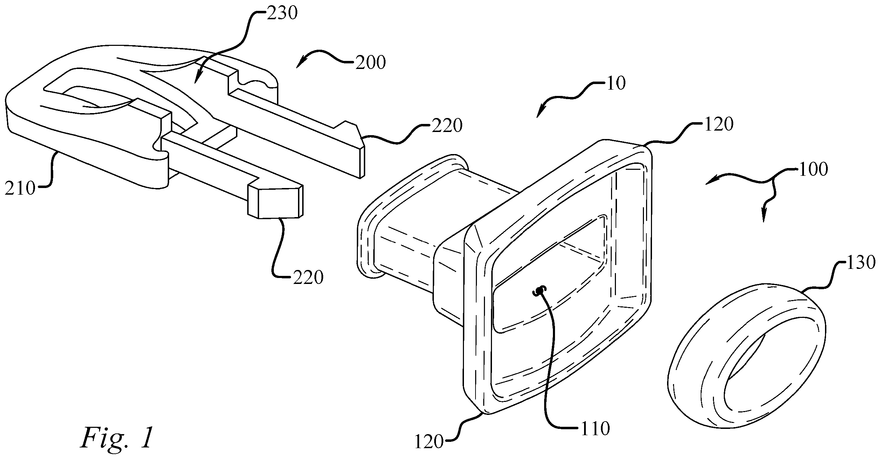

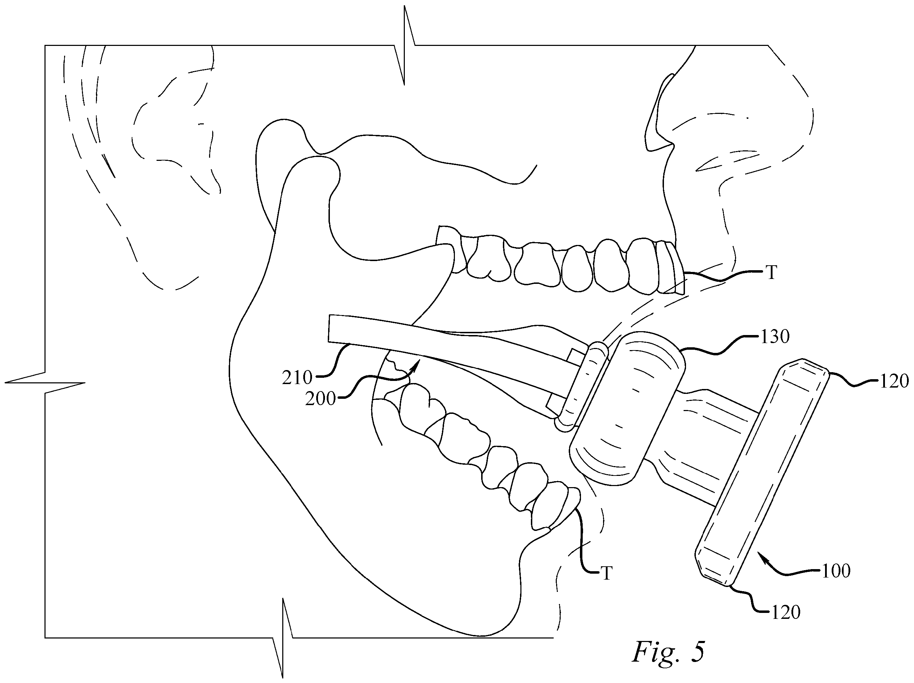

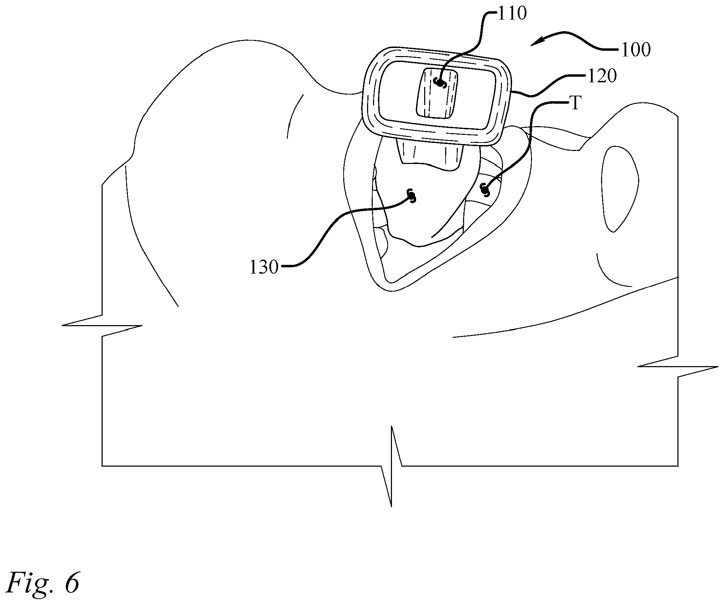

What is claimed then, as seen in FIGS. 1-10, is an immobilization system (10) for highly reproducible patient positioning. In the invention, seen well in FIGS. 1, 2, 7 and 9, the system (10) may include a bite block immobilization system (10) having a bite block (100) having a dental interface (130) releasably engageable with a plurality of teeth of a patient, and a bite block-retention mask interface (120) releasably engageable with a retention mask (400). The dental interface (130) may include a moldable dental impression putty in some embodiments, while in others, may include a moldable thermoplastic ring. The dental interface may be supplied with the system (10), or may be added at the time of use by a user. The dental interface (130) releasably engages the teeth of a patient and allows very highly accurate repositioning during multiple procedures.

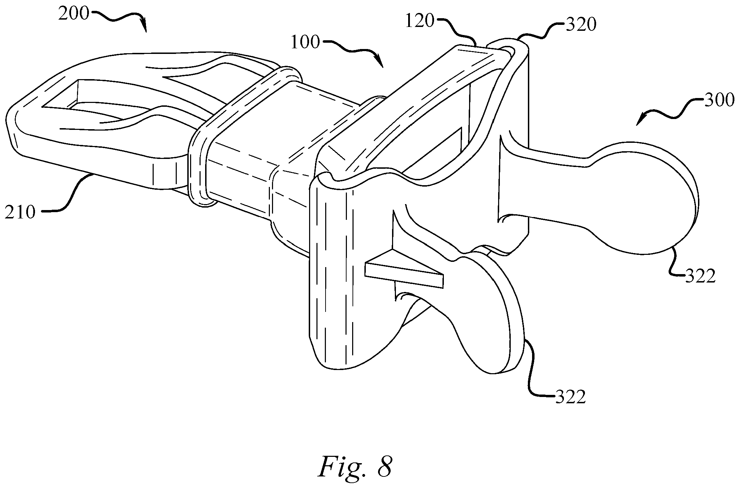

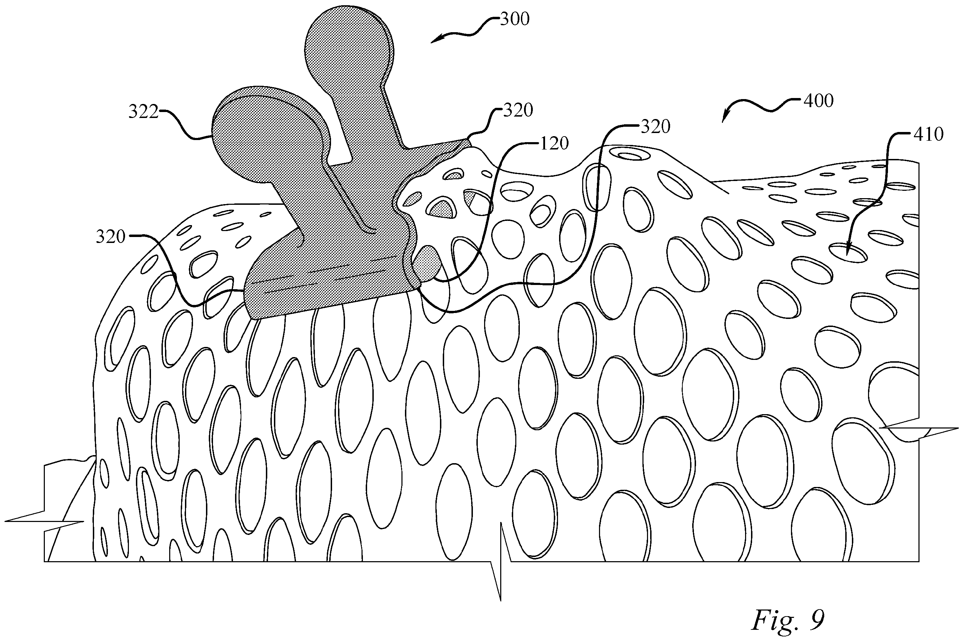

The retention mask (400), seen well in FIG. 9, is releasably and memorably moldable to the contours of a face of the patient. According to the invention, seen well in FIGS. 8 and 9, has a retainer means (300) cooperating with the bite block (100) and the retention mask (400) to releasably secure the bite block (100) and the retention mask (400). As would be known to one skilled in the art, the retention mask (400) may be secured to a backing board by any feasible releasable securing means for greater stability and reproducibility.

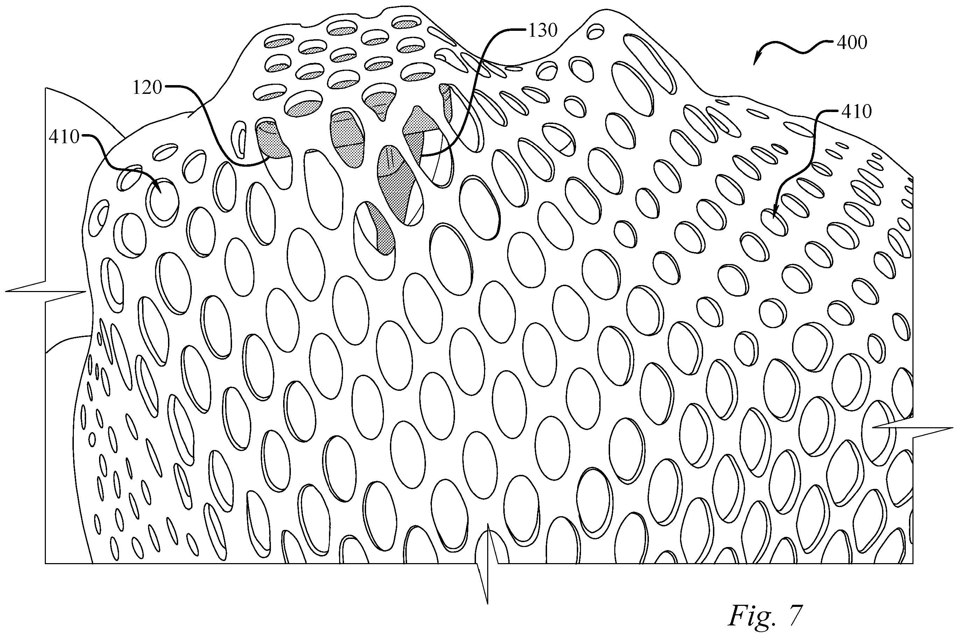

According to the invention, seen well in FIGS. 7 and 9, the retention mask (400) has a plurality of mask fenestrations (410). In other embodiments, seen well in FIGS. 1-3, the bite block (100) may have an integral bite block airway passage (110). This allows a patient to have unobstructed breathing both during molding of the retention mask (400) and during subsequent use of the system (10).

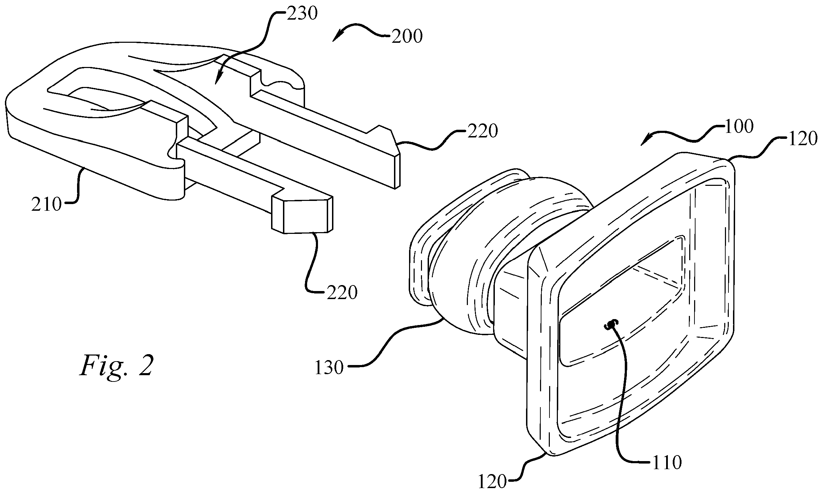

In the invention, the bite block (100), seen well in FIGS. 1-4 and 8, releasably cooperates at a tongue diverter receiver (140) with a tongue diverter (200), and, as seen well in FIGS. 2-3, the tongue diverter receiver (140) is releasably engageable with a tongue diverter retainer (220) to releasably engage the tongue diverter (200) and the bite block (100).

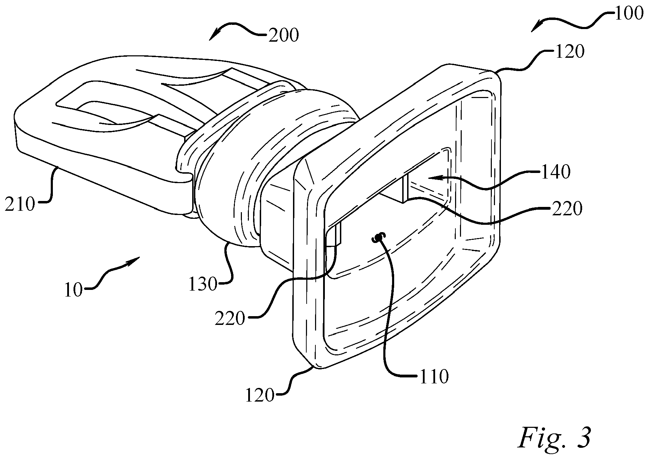

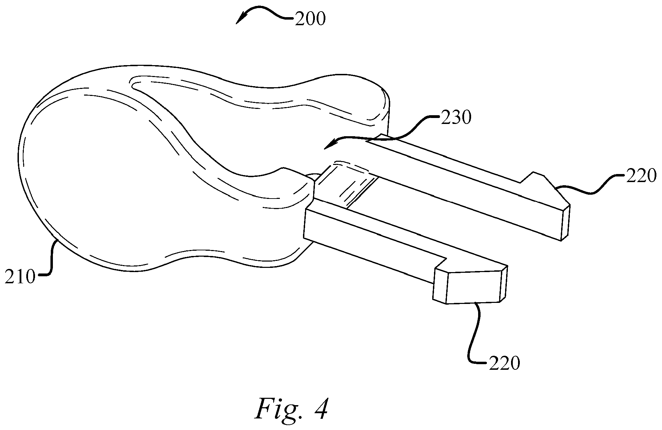

The tongue diverter (200) may further include as seen well in FIG. 4, a diverter airway (230) and includes, as seen well in FIGS. 3-5, a lingual interface (210). The lingual interface (200), as would be known by one skilled in the art, diverts the tongue of a patient in a lateral, cephalic, or caudal direction, or in some combination of multiple directions.

In the invention, seen by way of example and not limitation only in FIGS. 8-9, the retainer means (300) is releasably engageable with bite block (100) and the retention mask (400) by means of an interface retainer (320) releasable securing the bite block (100) to the retention mask (400). Such an interface retainer (320) is a humanly releasable digital manipulator (322). In the invention, seen well in FIGS. 10-11, a tongue diverter removal tool (240) is releasably engageable with the tongue diverter receiver (140) to releasably disengage the tongue diverter (200) and the bite block (100).

In another series of embodiments, a bite block immobilization system (10) may include a bite block (100) having an integral bite block airway passage (110), and a dental interface (130) releasably engageable with a plurality of teeth of a patient. The bite block (100) may be releasably cooperating at a tongue diverter receiver (140) with a tongue diverter (200) having a lingual interface (210) and a diverter airway (230). Such a tongue diverter receiver (140) may be releasably engageable with a tongue diverter retainer (220) to releasably engage the tongue diverter (200) and the bite block (100). Such embodiments may also include a bite block-retention mask interface (120) releasably engageable with a retention mask (400), and such a retention mask (400) may be being releasably and memorably moldable to the contours of a face of the patient. Further, there may be a retainer means (300) cooperating with the bite block (100) and the retention mask (400) to releasably secure the bite block (100) and the retention mask (400).

In the invention, the tongue diverter (200) further includes a lingual interface (210) that diverts the tongue of a patient in a lateral, cephalic, or caudal direction, or allow a diversion in multiple axes of direction. In other embodiments, the retainer means (300) releasably engageable with a bite block (100) and the retention mask (400) by means of an interface retainer (320) releasable securing the bite block (100) to the retention mask (400), and the interface retainer (320) is a humanly releasable digital manipulator (322). This obviates the need for a user to maintain pressure over the retention mask (400) while the retention mask (400) is converted from a more flexible, to a less flexible, state, most commonly by allowing the retention mask (400) to thermally cool.

One skilled in the art would recognize the most optimal methods of using the immobilization system. Steps for use might include; placing a bite block (100) between the maxilla and mandible of a human patient, and then placing a thermally moldable retention mask (400), in a moldable state, over at least a portion of the face of the human patient. Next, a user might secure the retention mask (400) to the bite-block (100) with a releasably engageable retainer means (300). Lastly, a user might observe or facilitate the conversion of the retention mask (400) to a fixed state, which could occur by any known method of converting the retention mask (400) from a flexible to a more rigid state, such as thermal hardening, and then, disengaging the releasably engageable retainer means (300).

Securing the releasable engageable retainer means (300) to the bite-block could be accomplished by the application of digital human pressure, and a subsequent step of may include the step of disengaging the releasable engageable retainer means (300) to the bite-block by the application of digital human pressure.

## Claims

1. A bite block immobilization system (10) comprising:
a bite block (100) having a dental interface (130) releasably engageable with a plurality of teeth of a patient, and a bite block-retention mask interface (120) releasably engageable with a retention mask (400);
the retention mask (400) being releasably and memorably moldable to the contours of a face of the patient, so that the retention mask (400) converts from a flexible to a more rigid state, such as by thermal hardening and
a retainer means (300) cooperating with the bite block (100) and the retention mask (400) to releasably secure the bite block (100) and the retention mask (400), wherein the retention mask (400) has a plurality of mask fenestrations (410), further wherein the bite block (100) releasably cooperates at a tongue diverter receiver (140) with a tongue diverter (200), wherein
- the dental interface (130) further comprises a dental impression putty or comprises a moldable thermoplastic ring, and wherein
- the retainer means (300) is releasably engageable with the bite block (100) and the retention mask (400) by means of an interface retainer (320) releasable securing the bite block (100) to the retention mask (400), wherein the interface retainer (320) is a humanly releasable digital manipulator (322), and wherein
- wherein a tongue diverter receiver (140) is releasably engageable with a tongue diverter retainer (220) to releasably engage the tongue diverter (200) and the bite block (100), and further wherein
- the tongue diverter (200) further comprises a lingual interface (210), wherein the lingual interface (200) adjustably diverts the tongue of a patient in a direction selected from a lateral, cephalic, caudal, and a combination of such directions, relative to the patient, and further wherein
- a tongue diverter removal tool (240) is releasably engageable with the tongue diverter receiver (140) to releasably disengage the tongue diverter (200) and the bite block (100.

2. A bite block immobilization system (10) according to the preceding claim, comprising:
a bite block (100) having an integral bite block airway passage (110), a dental interface (130) releasably engageable with a plurality of teeth of a patient, wherein the bite block (100) releasably cooperates at a tongue diverter receiver (140) with a tongue diverter (200) having a lingual interface (210) and a diverter airway (230), and wherein the tongue diverter receiver (140) is releasably engageable with a tongue diverter retainer (220) to releasably engage the tongue diverter (200) and the bite block (100);
a bite block-retention mask interface (120) releasably engageable with a retention mask (400), releasably and memorably moldable to the contours of a face of the patient, so that the retention mask (400) converts from a flexible to a more rigid state, such as by thermal hardening; and a retainer means (300) cooperating with the bite block (100) and the retention mask
(400) to releasably secure the bite block (100) and the retention mask (400), wherein
- the dental interface (130) further comprises a dental impression putty or comprises a moldable thermoplastic ring, and wherein
- the retainer means (300) is releasably engageable with bite block (100) and the retention mask (400) by means of an interface retainer (320) releasable securing the bite block (100) to the retention mask (400), wherein the interface retainer (320) is a humanly releasable digital manipulator (322), and wherein
- wherein a tongue diverter receiver (140) is releasably engageable with a tongue diverter retainer (220) to releasably engage the tongue diverter (200) and the bite block (100), and further wherein
- the tongue diverter (200) further comprises a lingual interface (210), wherein lingual interface (200) adjustably diverts the tongue of a patient in a direction selected from a lateral, cephalic, caudal, and a combination of such directions, relative to the patient, and further wherein
a tongue diverter removal tool (240) is releasably engageable with the tongue diverter receiver (140) to releasably disengage the tongue diverter (200) and the bite block (100).

## Patentansprüche

1. Ein Bissblock-Immobilisierungssystem (10), umfassend:
einen Bissblock (100) mit einer dentalen Schnittstelle (130), die lösbar mit einer Mehrzahl von Zähnen eines Patienten in Eingriff gebracht werden kann, und eine Bissblock-Retentionsmasken-Schnittstelle (120), die lösbar mit einer Retentionsmaske (400) in Eingriff gebracht werden kann;
wobei die Retentionsmaske (400) lösbar und erinnerbar an die Konturen eines Gesichts des Patienten anformbar ist, so dass die Retentionsmaske (400) von einem flexiblen in einen steiferen Zustand übergeht, beispielsweise durch thermische Härtung, und
einem Haltemittel (300), das mit dem Aufbissstück (100) und der Retentionsmaske (400) zusammenwirkt, um das Aufbissstück (100) und die Retentionsmaske (400) lösbar zu sichern, wobei
die Retentionsmaske (400) eine Vielzahl von Maskenfenstern (410) aufweist, wobei ferner
der Aufbiss (100) lösbar mit einer Zungenabweiser-Aufnahme (140) mit einem Zungenabweiser (200) zusammenwirkt, wobei
- die zahnärztliche Schnittstelle (130) ferner eine zahnärztliche Abdruckmasse oder einen formbaren thermoplastischen Ring umfasst, und wobei
- das Haltemittel (300) lösbar mit dem Aufbissstück (100) und der Retentionsmaske (400) mittels eines Schnittstellenhalters (320) in Eingriff bringbar ist, der das Aufbissstück (100) lösbar an der Retentionsmaske (400) befestigt, wobei der Schnittstellenhalter (320) ein von Menschenhand lösbarer digitaler Manipulator (322) ist, und wobei
- wobei eine Zungenabweiser-Aufnahme (140) lösbar mit einem Zungenabweiser-Halter (220) in Eingriff gebracht werden kann, um den Zungenabweiser (200) und den Aufbissblock (100) lösbar in Eingriff zu bringen, und wobei
- die Zungenumlenkung (200) ferner eine linguale Schnittstelle (210) umfasst, wobei die linguale Schnittstelle (200) die Zunge eines Patienten in einer Richtung, die aus einer lateralen, cephalen, kaudalen Richtung und einer Kombination solcher Richtungen ausgewählt ist, relativ zum Patienten einstellbar umlenkt, und ferner
ein Zungenabweiser-Entfernungswerkzeug (240) lösbar mit der Zungenabweiser-Aufnahme (140) in Eingriff gebracht werden kann, um den Zungenabweiser (200) und den Aufbiss (100) lösbar voneinander zu trennen.

2. Ein Bissblock-Immobilisierungssystem (10) gemäß dem vorstehenden Anspruch, umfassend:
einen Bissblock (100) mit einem integrierten Bissblock-Atemweg (110), eine Dentalschnittstelle (130), die lösbar mit einer Mehrzahl von Zähnen eines Patienten in Eingriff gebracht werden kann, wobei der Bissblock (100) lösbar mit einer Zungenumleitungsaufnahme (140) mit einer Zungenumleitung (20 0) mit einer lingualen Schnittstelle (210) und einem Umleitungs-Luftweg (230) zusammenwirkt, und wobei die Zungenumleitungs-Aufnahme (140) lösbar mit einem Zungenumleitungs-Halter (220) in Eingriff gebracht werden kann, um die Zungenumleitung (200) und den Aufbissblock (100) lösbar in Eingriff zu bringen;
eine Schnittstelle (120) zwischen Aufbissblock und Retentionsmaske, die lösbar mit einer Retentionsmaske (400) in Eingriff gebracht werden kann, die lösbar und einprägsam an die Konturen eines Gesichts des Patienten anformbar ist, so dass die Retentionsmaske (400) von einem flexiblen in einen steiferen Zustand übergeht, beispielsweise durch thermische Härtung; und ein Haltemittel (300), das mit dem Aufbissblock (100) und der Retentionsmaske (400) zusammenwirkt, um den Aufbiss (100) und die Retentionsmaske (400) lösbar zu sichern, wobei
- die zahnärztliche Schnittstelle (130) umfasst ferner eine zahnärztliche Abformmasse oder einen formbaren thermoplastischen Ring, und wobei
- das Haltemittel (300) lösbar mit dem Aufbissstück (100) und der Retentionsmaske (400) mittels eines Schnittstellenhalters (320) in Eingriff bringbar ist, der das Aufbissstück (100) lösbar an der Retentionsmaske (400) befestigt, wobei der Schnittstellenhalter (320) ein von Menschen lösbarer digitaler Manipulator (322) ist, und wobei
- wobei eine Zungenabweiser-Aufnahme (140) lösbar mit einem Zungenabweiser-Halter (220) in Eingriff gebracht werden kann, um den Zungenabweiser (200) und den Aufbissblock (100) lösbar in Eingriff zu bringen, und wobei
- die Zungenumlenkung (200) ferner eine linguale Schnittstelle (210) umfasst, wobei die linguale Schnittstelle (200) die Zunge eines Patienten in einer Richtung, die aus einer lateralen, cephalen, kaudalen Richtung und einer Kombination solcher Richtungen ausgewählt ist, relativ zum Patienten einstellbar umlenkt, und wobei ferner
ein Zungenabweiser-Entfernungswerkzeug (240) lösbar mit der Zungenabweiser-Aufnahme (140) in Eingriff gebracht werden kann, um den Zungenabweiser (200) und den Aufbiss (100) lösbar voneinander zu trennen.

## Revendications

1. Système d'immobilisation par bloc de morsure (10) comprenant :
un bloc de morsure (100) ayant une interface dentaire (130) pouvant être engagée de manière amovible avec une pluralité de dents d'un patient, et une interface bloc de morsure-masque de rétention (120) pouvant être engagée de manière amovible avec un masque de rétention (400) ;
le masque de rétention (400) peut être moulé de manière amovible et mémorisable aux contours du visage du patient, de sorte que le masque de rétention (400) passe d'un état souple à un état plus rigide, par exemple par durcissement thermique, et
un moyen de retenue (300) coopérant avec le bloc de morsure (100) et le masque de rétention (400) pour fixer de manière amovible le bloc de morsure (100) et le masque de rétention (400), dans lequel
le masque de rétention (400) comporte une pluralité de fenestrations de masque (410), en outre, dans lequel
le bloc de morsure (100) coopère de manière amovible au niveau d'un récepteur de déviation de la langue (140) avec un déviation de la langue (200), dans lequel
- l'interface dentaire (130) comprend en outre un mastic d'empreinte dentaire ou comprend un anneau thermoplastique moulable, et dans lequel
- le moyen de retenue (300) peut être engagé de manière amovible avec le bloc de morsure (100) et le masque de rétention (400) au moyen d'un dispositif de retenue d'interface (320) qui fixe de manière amovible le bloc de morsure (100) au masque de rétention (400), le dispositif de retenue d'interface (320) étant un manipulateur numérique (322) libérable par l'être humain, et dans lequel
- dans lequel un récepteur de déviation de langue (140) peut être engagé de manière amovible avec un dispositif de retenue de déviation de langue (220) pour engager de manière amovible le déviation de langue (200) et le bloc de morsure (100), et dans lequel en outre
- l'inverseur de langue (200) comprend en outre une interface linguale (210), dans laquelle l'interface linguale (200) détourne de manière réglable la langue d'un patient dans une direction choisie parmi les directions latérale, céphalique, caudale et une combinaison de ces directions, par rapport au patient, et dans lequel
- un outil d'extraction de l'inverseur de langue (240) peut être engagé de manière amovible avec le récepteur de l'inverseur de langue (140) pour désengager de manière amovible l'inverseur de langue (200) et le bloc de morsure (100).

2. Système d'immobilisation par bloc de morsure (10) selon la revendication précédente, comprenant :
un bloc de morsure (100) comportant un passage d'air intégré (110), une interface dentaire (130) pouvant être engagée de manière amovible avec une pluralité de dents d'un patient, dans lequel le bloc de morsure (100) coopère de manière amovible au niveau d'un récepteur d'inverseur de langue (140) avec un inverseur de langue (200) comportant une interface linguale (210) et un passage d'air d'inverseur (230), et dans lequel le récepteur de déviation de la langue (140) peut être engagé de manière amovible avec un dispositif de retenue de déviation de la langue (220) pour engager de manière amovible le déviation de la langue (200) et le bloc de morsure (100) ;
une interface bloc de morsure-masque de rétention (120) pouvant être engagée de manière amovible avec un masque de rétention (400), pouvant être moulé de manière amovible et mémorisable aux contours du visage du patient, de sorte que le masque de rétention (400) passe d'un état flexible à un état plus rigide, par exemple par durcissement thermique ; et un moyen de retenue (300) coopérant avec le bloc de morsure (100) et le masque de rétention (400) pour fixer de manière amovible le bloc de morsure (100) et le masque de rétention (400), de sorte que le masque de rétention (400) puisse être moulé de manière amovible et mémorisable.
(400) pour fixer de manière amovible le bloc de morsure (100) et le masque de rétention (400), dans lequel
- l'interface dentaire (130) comprend en outre un mastic d'empreinte dentaire ou comprend un anneau thermoplastique moulable, et dans lequel
- le moyen de retenue (300) peut être engagé de manière amovible avec le bloc de morsure (100) et le masque de rétention (400) au moyen d'un dispositif de retenue d'interface (320) fixant de manière amovible le bloc de morsure (100) au masque de rétention (400), le dispositif de retenue d'interface (320) étant un manipulateur numérique (322) libérable par l'homme, et dans lequel
- dans lequel un récepteur de déviation de langue (140) peut être engagé de manière amovible avec un dispositif de retenue de déviation de langue (220) pour engager de manière amovible le déviation de langue (200) et le bloc de morsure (100), et dans lequel en outre
- l'inverseur de langue (200) comprend en outre une interface linguale (210), dans laquelle l'interface linguale (200) détourne de manière réglable la langue d'un patient dans une direction choisie parmi les directions latérale, céphalique, caudale et une combinaison de ces directions, par rapport au patient, et dans lequel
un outil d'enlèvement du déviateur de langue (240) peut être engagé de manière amovible avec le récepteur du déviateur de langue (140) pour désengager de manière amovible le déviateur de langue (200) et le bloc de morsure (100).
